# EUROPÄISCHE PATENTSCHRIFT

(11) **EP 0 570 799 B1**
(45) Veröffentlichungstag und Bekanntmachung des Hinweises auf die Patenterteilung: **19.06.1996**
(21) Anmeldenummer: 93107560.0
(22) Anmeldetag: 10.05.1993
(51) Int. Cl.: C07C 263/10

(54) **Verfahren zur Herstellung von aromatischen Diisocyanaten**
Process for the preparation of aromatic diisocyanates
Procédé de préparation de diisocyanates aromatiques

(30) Priorität: 22.05.1992 DE 4217019
(43) Veröffentlichungstag der Anmeldung: 24.11.1993
(73) Patentinhaber: BAYER AG, 51368 Leverkusen (DE)
(72) Erfinder: Biskup, Klaus, Dr., W-5090 Leverkusen 1 (DE); König, Christian, Dr., W-4044 Kaarst (DE); Waldau, Eckart, Dr., W-4000 Düsseldorf 13 (DE)

(56) Entgegenhaltungen:
- EP-A- 0 289 840
- DD-B- 132 340

## Beschreibung

Die vorliegende Erfindung betrifft ein kontinuierliches Verfahren zur Herstellung von aromatischen Diisocyanaten durch Phosgenierung der entsprechenden Diamine, wobei die Umsetzung in der Gasphase durchgeführt wird.

Obwohl die Herstellung von organischen Isocyanaten aus den entsprechenden Aminen durch Umsetzung mit Phosgen in der Gasphase seit langem bekannt ist (z.B. Siefken, Ann. 562, 108 (1949)), war das Verfahren bisher nur für Monoamine (z.B. Ullmann, 4. Aufl. Bd. 13, S. 353) und (cyclo)aliphatische Diamine technisch bedeutsam. So gelingt die Herstellung (cyclo)aliphatischer Diisocyanate gemäß EP-A 289 840 in der Gasphase bei Temperaturen von 300 bis 500°C in einem Reaktionsrohr wahrend Reaktionszeiten von 10⁻⁴s. Aromatische Diamine dagegen wurden bislang stets in der Flüssigphase (z.B. Ullmanns, 4. Aufl. Bd. 13, S. 351) zu den entsprechenden Diisocyanaten phosgeniert. Die Gasphasenreaktion aromatischer Diamine mit Phosgen scheiterte an der Bildung von Feststoffen, die die Apparaturen verstopften und die Ausbeuten an Diisocyanaten herabsetzen.

Die Feststoffbildung ist sowohl zu langen als auch zu kurzen Reaktionszeiten zuzuschreiben. Zum Beispiel werden bei kurzen Reaktionszeiten neben Polyharnstoffstaub Isocyanataminhydrochloride gefunden. Bei zu langen Reaktionszeiten bilden sich feste Carbodiimide und Isocyanurate.

Es wurde nun gefunden, daß es dennoch gelingt, aromatische Diisocyanate unter Vermeidung von die Reaktoren verstopfenden Feststoffen bei Ausbeuten von über 95 %, im allgemeinen 99 % und höher, durch Gasphasenreaktion herzustellen, wenn der Kontakt zwischen Phosgen und aromatischen Diaminen innerhalb eines sehr eng begrenzten Kontaktzeitspektrums (Verweilzeitspektrum im Reaktor) gehalten wird.

Die notwendige Verweilzeit zur Reaktion beider Amingruppen mit dem Phosgen zu Isocyanat liegt zwischen 0,5 und 5 s, in Abhängigkeit von der Reaktionstemperatur, dem molaren Verhältnis von eingesetztem Amin und Phosgen, einer Verdünnung der gasförmigen Reaktionspartner mit Inertgasen und der Art des eingesetzten Diamins.

Wird die für das jeweilige System (Temperatur, molares Verhältnis, Verdünnungsgas, Diamin) einmal ermittelte Mindestverweilzeit für die vollständige Reaktion um weniger als etwa 20 %, vorzugsweise weniger als 10 %, überschritten, kann die Bildung von Weiter-Reaktionsprodukten wie Isocyanurate und Carbodiimiden wirksam vermieden werden.

Innerhalb dieses für chemische Reaktionen sehr engen Kontaktzeitspektrums muß sowohl die möglichst homogene Vermischung der Reaktionspartner als auch die weitere Reaktionszeit ohne Rückvermischung, die eine Verbreiterung des Kontaktzeitspektrums bewirkt, erfolgen.

Gegenstand der vorliegenden Erfindung ist demgemäß ein Verfahren zur Herstellung von aromatischen Diisocyanaten durch Umsetzung entsprechender Diamine mit Phosgen in der Gasphase, das dadurch gekennzeichnet ist, daß Phosgen und Diamin oberhalb des Siedepunkts des Diamins innerhalb einer mittleren Kontaktzeit von 0,5 bis 5 Sekunden, vorzugsweise 1,0 bis 3,7 Sekunden, umgesetzt werden, wobei die mittlere Abweichung von der mittleren Kontaktzeit weniger als 6 %, vorzugsweise weniger als 4 % betragen soll.

Das erfindungsgemäße Verfahren wird so durchgeführt, daß die Reaktionspartner oberhalb der Siedetemperatur des Diamins in geeignete Reaktoren eingeführt, vermischt und reagiert werden. Anschließend wird durch Abkühlung des Gasstromes das Isocyanat kondensiert, wobei die Abkühlung bis zu einer Temperatur oberhalb der Zersetzungstemperatur des entsprechenden Carbamidsäurechlorids erfolgt.

Bei der praktischen Durchführung des Verfahrens wird die Abweichung von der mittleren Kontaktzeit im wesentlichen durch die notwendige Zeit zur Vermischung der Reaktionspartner bestimmt. Solange die Reaktionspartner noch nicht homogen vermischt sind, sind im Reaktor noch unvermischte Gasvolumina vorhanden, die noch nicht zum Reaktionspartner in Kontakt treten konnten.

Die Vermischung der Reaktionspartner soll daher innerhalb einer Zeit 0,1 bis 0,3 Sekunden bis zu einem Segregationsgrad von 10⁻³ erfolgen. Segregationsgrad ist ein Maß für die Unvollständigkeit der Vermischung (siehe z.B. Chem.-Ing.-Techn. 44 (1972), S. 1051 ff; Appl. Sci. Res. (The Hague) A3 (1953), p. 279).

Die Methoden zur Durchführung kurzer Mischzeiten sind im Prinzip bekannt. Geeignet sind Mischaggregate mit bewegten oder statischen Mischorganen. Bevorzugt sind statische Mischorgane. Die Abhängigkeit des Segregationsgrades von der relativen Länge des Mischrohres (Verhältnis Länge der Mischstrecke zu Durchmesser) für einige statische Mischaggregate sind in Chemie-Ing.-Techn. 44 (1972) S. 1051-1056 dargestellt. Über die Länge der Mischstrecke läßt sich der für ein bestimmtes Mischaggregat notwendige Volumenstrom der Reaktionspartner zur Einhaltung der erfindungsgemäß zulässigen Mischzeit berechnen.

Besonders bevorzugt sind Strahlmischer. Hierbei wird in ein Mischrohr durch ein konzentrisches Rohr mit kleinem Durchmesser (Düse) mit hoher Geschwindigkeit die eine Komponente (hier Diamin) in den Strom der anderen Komponente (Phosgen) eingeblasen. Eine Vermischung mit Segregationsgrad 10⁻³ wird im Abstand des vierfachen Rohrdurchmessers vom Austrittsende der Düse erreicht (Chemie-Ing.-Techn. 44 (1972) S. 1055, Abb. 10). Konkrete Abmessungen für solche Strahlmischer lassen sich aus den verfügbaren experimentellen Daten grundsätzlich aufgrund der strömungsmechanischen Ähnlichkeitsgesetze berechnen.

Für die Erreichung eines möglichst hohen Segregationsgrades auf kurzer Mischstrecke ist auch das Impulsverhältnis der Gasströmungen im Strahlmischer wesentlich. Bei gegebenem Mischaggregat kann der Impuls des zentralen Gasstrahls (Diamin) durch Verdünnung mit einem in Bezug auf die Reaktion inerten Verdünnungsgas erhöht werden.

Bevorzugt wird als Verdünnungsgas Stickstoff eingesetzt. Geeignet sind ebenfalls Dämpfe von Chlorbenzol, o-Dichlorbenzol, Xylol, Chlornaphthalin, Decahydronaphthalin, oder ähnliche beziehungsweise deren Gemische. Das Volumenverhältnis von Verdünnungsmittel zu Diamin kann typischerweise zwischen 1:0,5 bis 1:2 liegen.

Nach Vermischung der Reaktionskomponenten durchströmt das Reaktionsgemisch das um den Reaktionsraum verlängerte Mischrohr zur Bereitstellung der restlichen Reaktionszeit. Die Durchströmung des Reaktionsraumes soll erfindungsgemäß in Form einer zu 90 % angenäherten Pfropfenströmung erfolgen, so daß alle Volumenteile der Strömung angenähert die gleiche Strömungszeit aufweisen, damit eine möglichst geringe weitere Verbreiterung der Kontaktzeitverteilung zwischen den Reaktionspartner erfolgt. Eine angenähert ideale Pfropfenströmung kann einerseits durch hochturbulente Strömungen mit Reynolds-Zahlen von oberhalb 4.000 im leeren Rohrreaktor realisiert werden.

Wegen der daher notwendigen hohen Strömungsgeschwindigkeiten ist die Realisierung der notwendigen Verweilzeit nur in sehr langen Misch- und Reaktorrohren möglich. Andererseits kann die Pfropfenströmung bei kleinerer Strömungsgeschwindigkeit auch durch Einbauten in das Reaktionsrohr realisiert werden, die der Ausbildung eines laminaren Strömungsprofils entgegen wirken und die Ausbildung einer ebenen Strömungsfront bewirken, Die Einbauten können z.B. die Form eines dreidimensionalen feinmaschigen Drahtgitters oder einer Füllkörperfüllung aufweisen.

Der Grad der Realisierung der idealen Pfropfenströmung (mit einer mittleren Abweichung von der mittleren Verweilzeit = 0) wird in der Strömungstechnik durch die Bodenstein-Zahl BO (Fitzer, Techn. Chemie, Springer 1989, 288 bis 295) beschrieben.

Erfindungsgemäß soll die Bodenstein-Zahl mindestens 100, vorzugsweise mindestens 250 betragen.

Bevorzugter Gegenstand der Erfindung ist demnach ein Verfahren zur Herstellung von Diisocyanaten der allgemeinen Formel

OCN-R-NCO

in welcher
- R: für einen Kohlenwasserstoffrest mit wenigstens einem aromatischen System steht, durch Phosgenierung der entsprechenden Diamine der allgemeinen Formel

H₂N-R-NH₂
in der Gasphase, das dadurch gekennzeichnet ist, daß man
a) die dampfförmigen Diamine, gegebenenfalls verdünnt mit einem Inertgas oder mit den Dampfen eines inerten Lösungsmittels, und Phosgen getrennt auf Temperaturen von 200 bis 600°C erhitzt und kontinuierlich in einer statischen Mischeinrichtung innerhalb von 0,1 bis 0,3 s bis zu einem Segrationsgrad von mindestens 10⁻³ vermischt,
b) das die Mischeinrichtung verlassende Reaktionsgemisch unter Vermeidung von Rückvermischung kontinuierlich durch einen auf 200 bis 600°C temperierten, rohrförmigen Reaktionsraum leitet und dort die Reaktion vollständig zu Ende führt, wobei die Strömung entweder durch eine Reynolds-Zahl von oberhalb 4.000 oder eine Bodenstein-Zahl von oberhalb 100 charakterisiert ist,
c) das aus dem Reaktionsraum austretende Gasgemisch zur Kondensation des gebildeten Diisocyanats abkühlt, wobei die Temperatur oberhalb der Zersetzungstemperatur des dem Diisocyanate entsprechenden Carbamidsäurechlorids gehalten wird,
d) nicht kondensiertes Diisocyanat aus dem Gasgemisch durch Waschen mit einem inerten Lösungsmittel abtrennt, und
e) das inerte Lösungsmittel in einer destillativen Aufarbeitung zurückgewinnt.

Das erfindungsgemäße Verfahren gestattet die Umsetzung von unzersetzt verdampfbaren, aromatischen Diaminen der allgemeinen Formel

H₂N-R-NH₂

in welcher
- R: für einen Kohlenwassestoffrest steht, der wenigstens ein aromatisches System enthält und mit weiteren Resten wie z.B. Alkylgruppen, Halogenatomen, Ethergruppen substituiert sein kann.

Die Aminogruppen der Diamine können beide mit ein und demselben aromatischen System im Kohlenwasserstoffrest R verknüpft sein oder auch an zwei verschiedene aromatische Systeme gebunden sein.

Typische Beispiele geeigneter Diamine sind die reinen Isomeren oder die Isomerengemische des Diaminobenzols, des Diaminotoluols, des Diamino-dimethylbenzols, sowie des Diamino-diphenylmethans. Bevorzugter Ausgangsmaterialien sind 2,4/2,6-Toluylendiamin-Gemische der Isomerenverhältnisse 65/35 und 80/20 oder das reine 2,4-Isomere.

Das bei der Phosgenierung verwendete Phosgen wird, bezogen auf das Diamin, im Überschuß eingesetzt. Im allgemeinen genügt eine Phosgenmenge, die 150 bis 250 % der Theorie bezüglich der ablaufenden Phosgenierungsreaktion entspricht.

Vor der Durchführung des erfindungsgemäßen Verfahrens wird der Phosgenstrom auf eine Temperatur innerhalb des Bereichs 200 bis 600°C, vorzugsweise 300 bis 400°C erhitzt.

Nach der erfolgten Phosgenierungsreaktion im Rohrreaktor wird das den Rohrreaktor kontinuierlich verlassende, gasförmige Gemisch von dem gebildeten Diisocyanat befreit, Dies kann beispielsweise einstufig durch selektive Kondensation in einem inerten Lösungsmittel erfolgen, wie es bereits für andere Gasphasenphosgenierungen empfohlen wurde (EP 0 289 840). Dabei wird die Temperatur so gewählt, daß sie einerseits oberhalb der Zersetzungstemperatur des dem Diisocyanat entsprechenden Carbamidsäurechlorids liegt und andererseits das Diisocyanat und gegebenenfalls das in dem Dampfstrom als Verdünnungsmittel mitverwendete Lösungsmittel kondensieren beziehungsweise sich in dem Lösungsmittel lösen, während überschüssiges Phosgen, Chlorwasserstoff und gegebenenfalls als Verdünnungsmittel mitverwendetes Inertgas die Kondensationsstufe beziehungsweise das Lösungsmittel gasförmig durchlaufen. Zur selektiven Gewinnung des Diisocyanats aus dem den Rohreaktor gasförmig verlassendenden Gemisch besonders gut geeignet sind bei einer Temperatur von 80 bis 200°C, vorzugsweise 80 bis 180°C gehaltene Lösungsmittel der oben beispielhaft genannten Art, insbesondere technisches Dichlorbenzol und Decahydronaphthalin.

Die Erzeugung der für das erfindungsgemäße Verfahren wesentlichen Strömung des gasförmigen Reaktionsgemisches als Pfropfenströmung ohne Rückvermischung ausgehend vom Mischaggregat durch den Rohrreaktor wird durch ein Druckgefalle zwischen den Produktzuleitungen zum Mischaggregat einerseits und dem Ausgang der Kondensationsstufe andererseits sichergestellt. Im allgemeinen liegt der Druck in den Zuleitungen zum Mischaggregat bei 200 bis 3000 mbar und hinter der Kondensationsstufe bei 150 bis 2000 mbar. Wesentlich ist hierbei jedoch lediglich die Aufrechterhaltung eines Differenzdrucks zwecks Gewährleistung der genannten gerichteten Strömung.

Das die Kondensationsstufe verlassende Gasgemisch wird anschließend in an sich bekannter Weise von überschüssigem Phosgen befreit. Dies kann mittels einer Kühlfalle, Absorption in einem bei einer Temperatur von -10°C bis 8°C gehaltenen inerten Losungsmittel (z.B. Chlorbenzol oder Dichlorbenzol) oder durch Adsorption und Hydrolyse an Aktivkohle erfolgen. Das die Phosgen-Rückgewinnungsstufe durchlaufende Chlorwasserstoffgas kann in an sich bekannter Weise zur Rückgewinnung des zur Phosgensynthese erforderlichen Chlors recyclisiert werden.

Die Reindarstellung der Diisocyanate erfolgt durch destillative Aufarbeitung der Lösungen aus der Kondensationsstufe.

In den nachfolgenden Beispielen beziehen sich alle Prozentangaben auf Gewichtsprozente.

### Beispiel 1

Durch eine Düse von 1 mm Innendurchmesser, die 20 mm in einen 500 mm langen Rohrreaktor von 25 mm Durchmesser hineinragt, strömt kontinuierlich ein in einem Wärmetauscher auf 420°C erwärmtes Gemisch von 0,5 mol/h einer gasförmigen Isomerenmischung von 2,4- und 2,6-Toluylendiamin (TDA) im Verhältnis 80 % zu 20 % mit 0,8 mol/h Stickstoff.

Durch den Ringspalt zwischen Düse und Reaktorwand strömen gleichzeitig 2,25 mol/h Phosgen, die ebenfalls auf 420°C vorgewärmt sind. 50 mm hinter der TDA-Düse beginnt eine Packung aus feinen Maschendrahtfüllkörpern, die den restlichen Teil des Reaktors ausfüllt. Der Rohrreaktor wird durch ein Heizbad mit einer Temperatur von 310°C von außen temperiert. Im Rohrreaktor hat das Reaktionsgemisch eine Verweilzeit von etwa 3 sec. Die Reynoldszahl beträgt etwa 210. Die Bodensteinzahl ist größer als 1000.

Das heiße, den Reaktor gasförmig verlassende Reaktionsgemisch wird durch eine Isocyanat-Absorptionskolonne geleitet, in der das Diisocyanat durch Verdampfung von im Gegenstrom geführten Dichlorbenzol kondensiert; das verdampfte Dichlorbenzol kondensiert in einem nachgeschalteten Rückflußkühler total und dient unterkühlt als Rücklauf der Isocyanat-Absorptionskolonne.

Das aus dem Rückflußkühler austretende Gasgemisch, das im wesentlichen aus Chlorwasserstoff, Phosgen und Stickstoff besteht, wird in einem anschließenden, mit Wasser berieselten Aktivkohlewaschturm von Phosgen und Chlorwasserstoff befreit.

Durch Anlegen eines Vakuums hinter dem Aktivkohlewaschturm wird am Ende des Rohrreaktors ein Druck von 800 mbar gehalten. Der Druck auf den Produktzuleitungen zur Mischkammer beträgt für das Gemisch aus Toluylendiamin-Dampf und Stickstoff 820 mbar, für das Phosgen 815 mbar.

Das gebildete Toluylendiisocyanat wird im Gemisch mit Dichlorbenol aus dem Sumpf der Isocyanat-Auswaschkolonne abgezogen und anschließend destillativ in reiner Form gewonnen. Die Ausbeute an Toluylendiisocyanat beträgt 99,3 %. Der Rohrreaktor zeigt keine Anzeichen von Verstopfung.

### Beispiel 2

Unter den Verfahrensbedingungn des Beispiel 1 werden 1,5 mol/h Toluylendiamin ohne Verdünnung mit Stickstoff mit 6,15 mol/h Phosgen umgesetzt. Die Verweilzeit im Rohrreaktor macht 1,2 sec aus. Die Reynoldszahl ist etwa 500. Die Bodensteinzahl ist etwa 1000.

Die Ausbeute an Toluylendiisocyanat beträgt 99,1 %.

### Beispiel 3

Unter den Verfahrensbedingungen des Beispiels 1 wird die Verweilzeit durch Verwenden eines Reaktors von nur 120 mm Länge wird auf unter 1 Sekunde verkürzt.

Die Ausbeute beträgt 98,3 %.

### Beispiel 4

Unter den Verfahrensbedingungen des Beispiel 1 werden 0,5 mol/h Toluylendiamin im Gemisch mit 0,8 mol/h Stickstoff mit 3,5 mol/h Phosgen zur Reaktion gebracht. Der Rohrreaktor enthält keine Füllkörper; die Verweilzeit beträgt etwa 2 sec. Die Reynoldszahl ist etwa 250, die Bodensteinzahl ist etwa 20.

Das Experiment wird nach 10 min abgebrochen, da Feststoffe die Isocyanatauswaschkolonne verstopfen.

### Beispiel 5

In der Apparatur des Beispiel 1 werden 0,5 mol/h 4,4'-Diamino-diphenylmethan im Gemisch mit 0,8 mol/h Stickstoff auf 410°C erhitzt und mit 3,5 mol/h Phosgen von 400°C zur Reaktion gebracht. Die Verweilzeit im Reaktor, der mit Füllkörpern gefüllt ist, beträgt 2,3 sec, der Reaktor wird mit einem Heizbad von 400°C temperiert. Der Druck am Ende des Reaktionsrohres beträgt 600 mbar. Die Kondensation erfolgt in Dichlorbenzol.

Die Ausbeute an Diphenylmethan-4,4'-diisocyanat beträgt 99,1 %.

## Patentansprüche

1. Verfahren zur Herstellung von aromatischen Diisocyanaten nach Umsetzung entsprechender Diamine mit Phosgen, dadurch gekennzeichnet, daß Phosgen und Diamin oberhalb der Siedetemperatur des Diamins innerhalb einer mittleren Kontaktzeit von 0,5 bis 5 Sekunden umgesetzt werden, wobei die mittlere Abweichung von der mittleren Kontaktzeit weniger als 6 % beträgt.

2. Verfahren nach Anspruch 1, dadurch gekennzeichnet, daß man
a) die dampfförmigen Diamine, gegebenenfalls verdünnt mit einem Inertgas oder mit den Dämpfen eines inerten Lösungsmittels, und Phosgen getrennt auf Temperaturen von 200 bis 600°C erhitzt und kontinuierlich in einer statischen Mischeinrichtung innerhalb von 0,1 bis 0,3 s bis zu einem Segregationsgrad von mindestens 10⁻³ vermischt,
b) das die Mischeinrichtung verlassende Reaktionsgemisch unter Vermeidung von Rückvermischung kontinuierlich durch einen auf 200 bis 600°C temperierten, zylindrischen Reaktionsraum leitet und dort die Reaktion vollständig zu Ende führt, wobei die Strömung entweder durch eine Reynolds-Zahl von oberhalb 4.000 oder eine Bodenstein-Zahl von oberhalb 100 charakterisiert ist,
c) das aus dem Reaktionsraum austretende Gasgemisch zur Kondensation des gebildeten Diisocyanats abkühlt, wobei die Temperatur oberhalb der Zersetzungstemperatur des dem Diamin entsprechenden Carbamidsäurechlorids gehalten wird,
d) nicht kondensiertes Diisocyanat aus dem Gasgemisch durch Waschen mit einem inerten Lösungsmittel abtrennt, und
e) das inerte Lösungsmittel in einer destillativen Aufarbeitung zurückgewinnt.

## Claims

1. Method for the preparation of aromatic diisocyanates by reaction of the corresponding diamines with phosgene, characterised in that phosgene and diamine are reacted above the boiling point of the diamine within an average contact time of from 0.5 to 5 seconds, with the average variation from the average contact time being less than 6%.

2. Method according to claim 1, characterised in that
a) the vaporous diamines, optionally diluted with an inert gas or with the vapours of an inert solvent, and phosgene are heated separately at temperatures of from 200 to 600°C and mixed continuously in a static mixer within a period of from 0.1 to 0.3 s to a degree of segregation of at least 10⁻³,
b) the reaction mixture leaving the mixer is passed continuously through a cylindrical reaction chamber heated at 200 to 600°C, with back-mixing being avoided, and there the reaction is taken to completion, with the flow being characterised by either a Reynolds number of above 4,000 or a Bodenstein number of above 100,
c) the gas mixture issuing from the reaction chamber is cooled in order to condense the diisocyanate formed, with the temperature being maintained above the decomposition temperature of the carbamic acid chloride corresponding to the diamine,
d) uncondensed diisocyanate is separated from the gas mixture by washing with an inert solvent, and
e) the inert solvent is recovered by distillation.

## Revendications

1. Procédé de préparation de diisocyanates aromatiques par réaction des diamines correspondantes avec le phosgène, caractérisé en ce que l'on fait réagir le phosgène et la diamine au-dessus de la température d'ébullition de la diamine avec une durée de contact moyenne de 0,5 à 5 s et un écart moyen de la durée de contact moyenne qui est inférieur à 6%.

2. Procédé selon la revendication 1, caractérisé en ce que
a) on chauffe séparément à des températures de 200 à 600°C les diamines à l'état de vapeur, éventuellement diluées par un gaz inerte ou par les vapeurs d'un solvant inerte, et le phosgène, et on mélange en continu dans un appareil de mélange statique en une durée de 0,1 à 0,3 s jusqu'à un degré de ségrégation d'au moins 10⁻³,
b) on envoie en continu le mélange de réaction quittant l'appareil de mélange, en évitant les mélanges en retour, au travers d'une chambre de réaction cylindrique chauffée à des températures de 200 à 600°C dans laquelle on achève la réaction, l'écoulement étant caractérisé soit par un nombre de Reynolds supérieur à 4000, soit par un nombre de Bodenstein supérieur à 100,
c) on refroidit le mélange gazeux sortant de la chambre de réaction pour condensation du diisocyanate formé en maintenant la température au-dessus de la température de décomposition du chlorure d'acide carbamique correspondant à la diamine,
d) on sépare le diisocyanate non condensé du mélange gazeux par lavage à l'aide d'un solvant inerte et
e) on récupère le solvant inerte par distillation.
